(19) Europäisches Patentamt
European Patent Office
Office européen des brevets

(11) Publication number: **0 405 315 B1**

(12) # EUROPEAN PATENT SPECIFICATION

(45) Date of publication of patent specification: **23.08.95**

(51) Int. Cl.6: **A61K 38/00**

(21) Application number: **90111644.2**

(22) Date of filing: **20.06.90**

(54) **Use of a phagocyte-activating agent : LPS or IL-2 for the manufacture of a medicament for treating staphylococcus aureus infection in cows.**

(30) Priority: **30.06.89 US 374339**

(43) Date of publication of application:
**02.01.91 Bulletin 91/01**

(45) Publication of the grant of the patent:
**23.08.95 Bulletin 95/34**

(84) Designated Contracting States:
**AT BE CH DE DK ES FR GB GR IT LI LU NL SE**

(56) References cited:
**EP-A- 0 097 580**
**EP-A- 0 215 576**
**EP-A- 0 271 270**
**EP-A- 0 281 380**
**EP-A- 0 285 441**
**EP-A- 0 359 873**

**Infection and Immunity, 55(3), 668-673, 1987**

(73) Proprietor: **AMERICAN CYANAMID COMPANY**
**One Cyanamid Plaza**
**Wayne, NJ 07470-8426 (US)**

(72) Inventor: **Daley, Michael Joseph**
**1564 Hummingbird Court**
**Yardly,**
**Pennsylvania 19067 (US)**

(74) Representative: **Wächtershäuser, Günter, Prof. Dr.**
**Patentanwalt,**
**Tal 29**
**D-80331 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 405 315 B1

## Description

### BACKGROUND OF THE INVENTION

The present invention relates to a composition for treating infectious diseases caused by Staphylococcus aureus bacterial infections in cows. Mastitis is an example of such a disease. The present method involves the establishment of therapeutic regime to optimize the effectiveness of phagocytic-activating agents, either alone or in combination with other therapies.

Animals' and plants' primary defense to acute and chronic diseases is through the mechanism known as phagocytosis. Disease-causing organisms or pathological cells cause in influx of phagocytic cells which identify and remove, by the host's phagocytic and immune system, those disease-causing organisms or pathological cells. The successful elimination of the pathogens or diseased cells involves a complex series of steps which activates phagocytic cells to optimize phagocytosis and intracellular or extracellular killing of the pathogen or diseased cell. Previously, the quantitative numbers of phagocytic cells have been shown to wax and wane in a cyclic response to many diseases (acute and chronic infections of the respiratory, urinary and hematopoietic systems for example). However, the present invention describes a method to therapeutically treat warm-blooded animals inflicted with a disease by monitoring and/or manipulating not only quantitative changes in phagocytic cells but qualitative changes in the cells during the disease process in a precise and predictable manner. These changes in phagocytic cells can be therapeutically affected by the administration of certain phagocytic-activating agents. Therefore, by the manipulation of this qualitative change in the phagocytic cell, a precise regulation and predicted therapeutic remedy for the treatment of the disease is available for the first time.

The present invention relates to compositions for treating infectious diseases to effect a cure by provoking or eliciting a 10 to 10,000 fold activation of normal phagocytosis and intracellular killing of pathogens or diseased cells. A similar activation occurs during normal pathophysiology and at predicted periods during diseases. It is a sustained change in the activation state of the phagocytic cells, not the numbers, that is the important feature in eliciting a method for treating infectious diseases that exhibit high and low stages of cellular activation. Therefore, any phagocytic-activating agent capable of provoking or eliciting the appropriate activation is an effective therapeutic agent. Thus, appropriate dosage and timing of therapy in manipulating the phagocytic-activating response is central to a predictive and effective treatment of such diseases. The present invention, for the first time, provides such a method and provides a method for enhancing the therapeutic efficacy of non-phagocytic-cell-activating agents, by combining the phagocytic-cell-activating agents of the present invention with those cells.

### BRIEF DESCRIPTION OF DRAWINGS

Figure 1. Four infected mammary glands, panels A, B, C, and D, from two animals are followed for five consecutive days. Both somatic cell counts, SCC, and colony forming units, CFU, of Staphylococcus aureus in milk are quantitated on each day. Data is expressed as the mean SCC X $10^3$ or the number of CFU/0.1 ml plated.

Figure 2. Mammary glands are infused with compounds which exhibit possible chemoattractant properties. Somatic cells are quantitated from mammary secretions at various times after intramammary infusion using a Coulter counter (Model ZM & C256 Channelyzer, Coulter Electronics, Northwell Dr., Luton Beds, England). Cellular response to the chemoattractant is expressed as the stimulation index ({post-infusion cell count} ÷ {pre- infusion cell count}).

Figure 3. Mammary glands are infused with a chemoattractant compound, such as lipopolysaccharide (0.1 μg to 25 μg total dose), and somatic cells are quantitated from mammary secretions at 12, 24, 36 and 48 hours after intramammary infusion using a Coulter counter (Model ZM & C256 Channelyzer, Coulter Electronics, Northwell Dr., Luton Beds, England). Cellular responses to the chemoattractant are expressed as the stimulation index ({post-infusion cell count} ÷ {pre-infusion cell count}). The peak and duration of the response is adjusted to be optimal for the individual disease state being treated.

Figure 4. Various chemoattractants are used to induce an influx of somatic cells into the milk. At 8, 24, 36, 48 and 72 hours after infusion the somatic cells are counted in the milk. In addition, the average size of the induced population is evaluated on a Coulter Channelyzer (C256 Channelyzer, Coulter Electronics, Northwell Dr., Luton Beds, England). The diameter of the peak population is plotted vs. the time after infusion. The normal uninfected gland somatic cell population has a cell diameter at the peak of 8.3μm.

Figure 5. Phagocytic cells are incubated with 2m fluorescent beads for 1 hr. at 37°C. The relative degree of fluorescence is then quantitated using a flow cytometer (EPICS 753, Coulter Electronics, Hialeah, FL). The percentage of cells engulfing 0, 1, 2, 3, or 4+ beads is calculated on the basis of fluorescence intensity.

Figure 6. Recombinant bovine interleukin-2, r-BoIL-2, is administered through a single intramammary infusion at 0.4 mg to 40 mg in a 10 ml total dose of phosphate buffered saline into mammary quarters of normal Holstein dairy cattle. The somatic cell count is monitored at 24 hours after infusion by Coulter count (Model ZM & C256 Channelyzer, Coulter Electronics, Northwell Dr., Luton Beds, England). The Stimulation Index is calculated by dividing the pre-infusion somatic cell count by the post-infusion somatic cell count.

Figure 7. Recombinant bovine interleukin-2, r-BoIL-2, is administered through a single intramammary infusion of 2 mg in 10 ml total dose into mammary quarters of Staphylococcus aureus infected Holstein dairy cattle. The phagocytic index of isolated somatic cells is monitored at 0, 16, 24, 40 and 64 hours after infusion by the ingestion of fluorescent beads and quantitated by flow cytometry (EPICS 753, Coulter Electronics, Hialeah, FL). Data is expressed as the the total percent of the polymorphonuclear population of somatic cells ingesting beads ± standard deviation of three different samples.

Figure 8. Phagocytic cells isolated from individual mammary quarters over 64 hrs. treated with 2 mg of r-BoIL-2 are incubated with 2$\mu$ fluorescent beads for 1 hr. at 37°C. The relative degree of fluorescence is then quantitated using a flow cytometer (EPICS 753, Coulter Electronics, Hialeah, FL). The percentage of cells engulfing 0, 1, 2, 3, or 4+ beads ± standard deviation is calculated on the basis of fluorescence intensity from samples from three different animals.

Figure 9. Recombinant bovine interleukin-2, r-BoIL-2, is administered thrice every 24 hours or every 48 hours through an intramammary infusion at 2 mg to 10 mg in 10 ml total dose into mammary quarters of Staphylococcus aureus infected Holstein dairy cattle. The phagocytic index of isolated somatic cells is monitored over a 40 hour period by the ingestion of fluorescent beads and flow cytometry (EPICS 753, Coulter Electronics, Hialeah, FL). Data is expressed as the the total percent of the polymorphonuclear population of somatic cells ingesting beads.

Figure 10. Recombinant bovine interleukin-2, r-BoIL-2, is administered thrice every 24 hours or every 48 hours through an intramammary infusion at 2 mg in 10 ml total dose into mammary quarters of Stauhylococcus aureus infected Holstein dairy cattle. The phagocytic index of isolated somatic cells is monitored at 16 and 40 hours after intramammary infusion by the ingestion of fluorescent beads and quantitated by flow cytometry (EPICS 753, Coulter Electronics, Hialeah, FL). Data is expressed as the the total percent of the polymorphonuclear population of somatic cells ingesting 1, 2, 3, or 4+ beads ± standard deviation.

## SUMMARY OF INVENTION

The present invention provides the use of a composition for the manufacture of a medicament for treating infectious diseases by Staphylococcus aureus in cows as claimed in Claim 1.

With this composition the phagocytic cell response in cows is manipulated through the use of phagocytic-activating agents. One of the diseases mentioned above is mastitis, found in cows.

## DETAILED DESCRIPTION OF THE INVENTION

The present invention relates to methods for treating animals with infectious diseases by enhancing the phagocytic cell response elicited by administering a phagocytic-activating amount of a phagocytic-activacting agent. The following examples related specifically to Staphylococcus aureus bacterial infections of the mammary tissue, but it is recognized to those of ordinary skill in the art that the present method of treating infectious diseases applies to any disease in which a cyclic phagocytic-activation response is present.

The present invention also provides an effective method for preventing diseases such as mastitis by utilizing the compounds eliciting the phagocytic-activating response. More specifically, mastitis is prevented by administering a mastistis-preventing amount of bovine interleukin-2 (bIL-2).

Example 1

## Staphylococcus aureus Infections in Mammary Tissue of Cows

Milk samples are collected every 24 hours from glands experimentally infected with Staphylococcus aureus (the American Type Culture strain Newbould 305 is preferred) for a minimum of three weeks. A total of 100μl of milk is plated on a blood agar plate and incubated for 18 hrs. at 37°C. The number of β-hemolytic colonies is determined and is expressed as bacterial load per ml of sample. Similarly, 1 ml of sample also is evaluated for the number of viable somatic cells. Greater than 90% of the somatic cells in an infected gland are phagocytic cells as morphologically characterized.

The cycling of somatic cells is inversely related to the quantitative changes in bacteria in the milk. The rise in somatic cells (primarily phagocytic cells) corresponds with a decrease in the bacterial count in the milk. Conversely, as the somatic cell count decreases, the bacterial count eventually increases and reestablishes the infection, Figure 1A, B, C, and D. This cyclic rise and fall of infections is also known to occur during the response by hosts to various other disease states, such as acute and chronic respiratory or urinary system infections, infections of the gastrointestinal tract, chronic inflammatory bowel diseases, infections of skin, autoimmune diseases (rheumatoid arthritis, multiple sclerosis, systemic lupus erythematosis, etc.), inflammatory responses to parasitic infections and responses to tumors (especially lymphomas, leukemias, melanomas and other solid tumors).

Example 2

Qualitative Changes in Phagocytic Cells

Milk samples from infected glands are collected and the somatic cells pelleted and counted. These cells are extensively washed and then treated with a solution of lysostaphin to eliminate Staphylococcus aureus adhered to the surface but not phagocytosed. The phagocytic cells are then lysed through repetitive freeze thawing in a water solution. The lysed solution is then plated on a blood agar plate for 18 hours at 37°C, and viable β-hemolytic colonies are quantitated as a measure of internal viable Staphylococcus aureus. Since the number of somatic cells in the pellet that is lysed is known, the frequency of phagocytic cells with a viable internal Staphylococcus aureus can be quantitated, i.e. intracellular killing failures.

The frequency at which cells are identified which harbor viable pathogens or fail to kill pathological cells, i.e. killing failures, changes during the infection, see Table I.

TABLE I

INTRACELLULAR KILLING OF STAPHYLOCOCCUS AUREUS

| Day/Source | Somatic Cells # Cells/ml (X $10^4$) | External S.aureus CFU/ml (X $10^1$) | Relative Efficiency # Cells/CFU (X $10^4$) |
|---|---|---|---|
| Day 1 - LF[1] | 17.3 | 71 | 0.1[2] |
| Day 1 - LR | 25.3 | 113 | 0.6 |
| Day 1 - RF | 23.8 | 19 | 1.2 |
| Day 1 - RR | 74.5 | 60 | >7.5 |
| Day 2 - LF | 45.9 | 12 | 19.1 |
| Day 2 - LR | 32.0 | 9 | 71.2 |
| Day 2 - RF | 6.0 | 19 | >30.3 |
| Day 2 - RR | 15.2 | 3 | 25.3 |
| Day 3 - LF | 630.0 | 115 | 3150 |
| Day 3 - LR | 580.0 | 1 | 2900 |
| Day 3 - RF | 220.0 | 8 | 1470 |
| Day 3 - RR | 430.0 | 1 | 4300 |

1 LF = left fore quarter of the mammary gland; LR = left rear quarter of the mammary gland; RF = right fore quarter of the mammary gland; RR = right rear quarter of the mammary gland.

2 The relative efficiency of killing of Staphylococcus aureus by different populations of PMN's from an infected gland. This is calculated by calculating the number of cells required to be lysed in order to detect a viable Staphylococcus aureus. The lower the number the less efficient the cells were at eliminating the Staphylococcus aureus.

This variation in efficiency of killing intracellular Staphylococcus aureus is as much as 10,000 fold. Cells which do not efficiently 'kill' the pathogen contribute to the reinfection of the host by protecting them from therapies (antibiotics, etc.) which cannot penetrate the host's cells. These inefficient cells die and release their viable pathogens or pathological cell targets. It is these cells which are the targets of the phagocytic-activating agents for therapy. Methods or substances which activate phagocytic cells to their optimal biological functional capacity, as measured by methods described herein, and at the appropriate time, thereby maximize efficiency and minimize release or escape of pathogens or pathological cells and optimize therapeutic efficacy. In addition, excess or untimely activation can lead to suppression of biological activity. Therapy inappropriately targeted can in fact hinder the normal host's defense mechanism. Although qualitative and/or quantitative changes in phagocytes has been described, the fact that these fluctuations are coordinated, synchronous and definable, allows the identification of appropriate therapies and therapeutic regimes.

Example 3

Chemoattractant Effects

A number of chemoattractants (compounds and/or biologicals which directly or indirectly cause a specific migration of cells towards a higher concentration gradient containing them), often endogenous to pathogens or diseased cells, induce an influx of phagocytic cells if locally or systemically administered. This influx of phagocytic cells in part mimics the normal cycling influx of phagocytic cells in an infected area. Normal pathogen 'free' mammary glands are infused through the teat canal with various known chemoattractants. The dose of these chemoattractants also is varied and the influx of cells into the mammary gland monitored by determining the somatic cell count from milk collected at various times after challenge. The number of somatic cells is determined on a Coulter Counter (Model ZM & C256 Channelyzer, Coulter Electronics, Northwell Dr., Luton Beds, England). The post-infusion cell count ÷ pre-infusion cell count gives a relative measure of the degree of stimulation by the chemoattractant, expressed as a Stimulation Index.

Most of the potential chemoattractants cause a significant influx of phagocytic cells into the mammary gland, Figure 2. However, the dose of administration of these chemoattractants not only determines the degree of stimulation but also determines the time to a peak response and/or longevity of the elevated numbers of phagocytic cells, Figure 3. Therefore, chemoattractant therapy is determined by altering the dose (0.1 $\mu$g to 100 mg, or up to 100% of active compound, such as zymosan activated cow serum, ZACS is preferred) to elicit a specific peak response and duration to coordinate with the cycling disease state.

Example 4

Testing of Chemoattractants

As with a normal cycling response to a disease state, phagocytic cells induced by various chemoattractants are qualitatively different. Cells induced by chemoattractants as provided for in Example 3 are evaluated for their mean cell diameter using a Coulter Counter and Channelyzer (Model ZM & C256 Channelyzer, Coulter Electronics, Northwell Dr., Luton Beds, England). The mean of the cell diameter of the somatic cell population peak from 3-4 individual challenged glands are calculated at 8, 24, 36, 48 and 72 hours after challenge, Figure 4. Some chemoattractants elicit differential changes in cell diameter with time (lipopolysaccharide and zymosan activated bovine sera, for example), while others have no effect (protein A, for example) and the mean cell diameter remains relatively constant. These coordinate changes in cell size also predict levels of activation of these phagocytic cells. The same doses which elicit distinct qualitative changes in phagocytosis and activation elicit a similar quantitative influx of phagocytic cells. Fluctuations in the cell diameter of the peak of the population of greater than 10% greater peak diameter of unstimulated control cell populations during a 12-72 hour period after intramammary infusion can be indicative of appropriate activation of cells.

Example 5

Classes of Phagocytic-Activating Compounds

As provided in Example 4, normal mammary glands are infused with lipopolysaccharide, LPS, or zymosan activated cow serum, ZACS. Doses ranging from 0.5 - 50 $\mu$g for LPS and 10 ml of 1% to 100% ZACS are preferred. The biological activity of phagocytosis is evaluated by measuring the ability of induced or non-induced cells to phagocytose fluorescenated 2$\mu$ beads. The percent of cells phagocytosing beads, as well as the mean number of beads phagocytosed by each cell, is quantitated by flow cytometry (EPICS 753, Coulter Electronics, Hialeah, FL), Figure 5.

Cells from non-induced glands or glands induced with 1 $\mu$g lipopolysaccharide or 10 ml of zymosan activated bovine sera are assayed at 24 hours post-infusion for their ability to phagocytose fluorescenated beads. Even though the quantitative induction by these two chemoattractants is similar, the lipopolysaccharide-induced population has a greater percentage of cells phagocytosing beads, 45% vs. 30%, as well as a higher percentage of cells phagocytosing more than one bead, 21-28% vs. 15-16%, Table II.

TABLE II
PHAGOCYTOSIS OF FLUORESBRITE BEADS BY BOVINE PMN'S

% PHAGOCYTOSIS OF TOTAL POPULATION[1]

| | Number of Beads Ingested | | | | |
|---|---|---|---|---|---|
| Cell Source | 1 | 2 | 3 | 4 | Total |
| Normal (Unstimulated) | | | | | |
| 30 min | 18.64 | 4.64 | 1.87 | | 25.15 |
| 1 hr | 20.80 | 6.23 | | | 27.03 |
| 2 hr | 21.87 | 9.62 | 3.67 | | 35.16 |
| 1µg LPS | | | | | |
| 30 min | 19.47 | 12.40 | 9.00 | 6.26 | 47.13 |
| 1 hr | 20.38 | 12.58 | 8.67 | 5.94 | 47.57 |
| 2 hr | 17.93 | 9.89 | 6.13 | 4.89 | 38.84 |
| 10ml Zymosan Activated Sera | | | | | |
| 30 min | 13.64 | 8.85 | 7.31 | | 29.80 |
| 1 hr | 15.23 | 9.23 | 6.36 | | 30.82 |

1 The total population was first gated by the computer to only consider the cells, polymorphonuclear cells (PMN's), which were of the proper size (forward angle light scatter, FALS, and internal cellular structure ($90^{\circ}$ light scatter). Therefore, quantitation of phagocytosis (ingestion of fluorescent beads) was measured on viable PMN's.

A range of ingestion of fluorescent beads of 10 % to 100 % phagocytosis above control levels results in effective therapeutic treatment of said disease. In addition, proper activation also involves the increase in the number of fluorescent beads ingested per cell by as much as 1.5-4 fold. Lipopolysaccharide is an example of a phagocytic cell activating compound which induces a quantitative influx of cells (greater than 10-20 fold increase in somatic cells in the milk over the untreated normal control quarters) and concomitantly activates the cells as well, whereas the zymosan activated bovine sera induces an influx of phagocytic cells (greater than 10-20 fold increase in somatic cells in the milk over untreated normal control quarters) into the gland without significantly activating them.

A summary of representative examples of chemoattractants are similarly analyzed for time of peak influx and activation of phagocytosis, Table III.

TABLE III

SUMMARY OF PROPERTIES OF CHEMOATTRACTANTS

| Chemoattractant | Concentration | QUANTITATIVE (SCC)[1] 12 hr | 24 hr | 36 hr | QUALITATIVE[2] Phagocytosis | Class |
|---|---|---|---|---|---|---|
| Lipopolysaccharide(LPS) | 1 µg/ml | + | ++ | + | +++ | I |
| Lipopolysaccharide | 5 µg/ml | ++ | +++ | + | ++++ | I |
| Lipopolysaccharide | 25 µg/ml | ++++ | +++ | + | ND | I |
| Enterotoxin B | 25 µg/ml | +++ | ND | + | ND | I |
| Protein A | 25 µg/ml | + | ++ | + | + | II |
| Zymosan | 10 % | ++ | +++ | ++ | + | II |
| Oyster Glycogen | 100 mg/ml | +/- | + | +/- | ND | II |
| F127 | 15 % | + | ++ | + | ND | II |
| Lipid A of LPS | 10 µg/ml | - | - | - | ND | - |
| Polysaccharide of LPS | 10 µg/ml | - | - | - | ND | - |

1 Somatic cell count, compared to untreated quarter.

2 Relative phagocytosis of fluorescenated beads as compared to controls.

Two distinct classes exist. Class I predicts compounds which serve as chemoattractants and chemoactivators (compounds which increase the biological activity of cells, particularly for phagocytic cells the ingestion and elimination of foreign material). Class II includes compounds which primarily act as chemoattractants with minimal or no activation. The dose of each class of chemoattractants controls the time to peak response and the duration of the response. The preferred dose being lipopolysaccharide (LPS), enterotoxin B, protein A, 0.5 - 50 μg; zymosan activated cow sera, ZACS, F127 pluronic, 10-50 μl of 1-50% solution; oyster glycogen, 5-200 mg; lipid A of LPS, 1-50 μg; and polysaccharide of LPS, 1-10 μg. If therapy for a disease state should require influx of cells as well as activation, then representatives of Class I chemoattractants provide the best therapeutic choice. The dose controls the degree and length of response to be optimized for the cycling infection, as in Example 3.

Example 6

Therapeutic Efficacy of Classes of Phagocytic-Activating Compounds

As provided in Example 4, representatives from both classes of chemoattractants/chemoactivators are used to treat chronic bacterial infections. The effectiveness of the therapy is predicted by cures and/or the length of time the infection remains cleared, Table IV. The Class I compounds are the most effective with this therapeutic regime, Figure 5.

TABLE IV

CHEMOATTRACTANT/CHEMOACTIVATOR THERAPY OF CHRONIC INFECTIONS

| Treatment | # of Mammary Glands | % Cures | % Responding | # of Clear Milkings +/- SEM[1] |
|---|---|---|---|---|
| LPS[2] | 11 | 18 (2) | 100[3] | 5.2 +/- 4.3 |
| ZACS[4] | 7 | 0 | 0 | 0.0 +/- 0.0 |

1 Number represents the mean time of Staphylococcus aureus free milkings +/- the standard error of the mean, SEM.

2 Treatment used 1 µg LPS in a total volume of 10 ml of phosphate buffered saline infused into the infected mammary gland on three day intervals.

3 Percent of treated glands which demonstrated a bacteriological clearing of the infection for at least one milking after therapy.

4 Treatment used 10 ml of (100%) Zymosan activated cow sera, ZACS, infused on three consecutive days into the infected area.

Example 7

Quantitative Cellular Response to a Phagocytic-Activating Cytokine

As in Examples 3 and 4 the r-BoIL-2 cytokine is infused into the mammary gland of uninfected dairy cattle. The somatic cell count is monitored every 8-16 hours after infusion and the Stimulation Index calculated as described in Example 3. Recombinant bovine IL-2 causes an immediate (8-16 hours) and dramatic influx (as much as 3000 fold over control levels) of primarily polymorphonuclear cells (>90% by morphology and cellular differentiation antigen expression). A second wave of cellular infiltration into the mammary gland occurs at 36-72 hours after administration. The degree of quantitative cellular influx is approximately related to the dose of r-BoIL-2 administered, Figure 6. This massive infiltration of polymorphonuclear cells, a cell type which is not known to express specific receptors for the cytokine IL-2 is new and unexpected.

Example 8

Qualitative Cellular Reponses to a Phagocytic-Activating Cytokine

Somatic cells, greater than 90% polymorphonuclear cells, from mastitic animals are evaluated for their ability to phagocytose 2μ fluorescent latex beads after single infusion of recombinant bovine interleukin-2, r-BoIL-2. Cells from the single administration of 2 mg r-BoIL-2 are incubated with beads at 16, 24, 40 and 64 hours after infusion. Cells from animals administered 2 mg of r-BoIL-2 thrice at 24 or 48 hour intervals are tested for phagocytic activity 16 and 40 hours after the last administration. The samples are then analyzed and phagocytosis quantitated by flow cytometry.

Infected mammary quarters are treated with r-BoIL-2, at 2, 10 and 30 mg in 10 ml sterile PBS. Pre-treatment samples are collected to evaluate phagocytic activity and establish a baseline. Post-treatment samples are collected at regular intervals for up to seven (7) days, and their phagocytic ability and intracellular killing determined as described. A minimum of 10% increase in phagocytosis is observed in all quarters appropriately treated. In Figure 7 the single dose of 2 mg of r-BoIL-2 shows a 3.5 fold increase in phagocytic activity of cells at 64 hours after initial infusion. In addition, an increase in the average number of beads ingested per cell is also observed as a measure of increased biological activity, Figure 8.

The ability of milk somatic cells from mastitic glands to phagocytose latex beads is measured from samples collected 16 and 40 hours after the last administration of r-BoIL-2 (either 2 mg or 10 mg dose is preferred). The r-BoIL-2 is administered thrice at 24 hour or 48 hour intervals. In Figure 9, the total percent of the polymorphonuclear cells that phagocytosed beads is compared for the two regimens at the 2 mg dose.

A three to four fold enhancement of phagocytic ability is observed with these treatments. Similarly, as an indicator of cellular activation after infusion of 2 mg r-BoIL-2, a significant shift in the number of cells ingesting 2 or more beads is observed, Figure 10.

Example 9

As provided in Example 4 and 8, r-BoIL-2 is infused into the mammary gland to treat chronic bacterial infections. The effectiveness of the therapy is predicted by cures and/or the length of time the infection remains cleared. As provided in Example 7 and 8 the quantitative and qualitative changes induced by r-BoIL-2 place this cytokine in the Class I type of compounds and are predicited to be an effective therapeutic as provided in Table V.

As in Example 8, infected mammary glands are treated with an intramammary infusion of r-BoIL-2 (preferred dose of 2-40 mg administered three times at 24-48 hour intervals). The milk is collected for 14 successive days in the morning and examined for the presence of Staphylococcus aureus (b-hemolytic colonies on blood agar plates). Mammary quarters having any milk samples free of Staphylococcus aureus are responding to therapy. Mammary quarters remaining free of Staphylococcus aureus for 14 days are cured. The r-BoIL-2 therapy, causes quantitative and qualitative changes in phagocytic cell function and is therefore efficacious as a therapeutic, Table V.

TABLE V

CYTOKINE THERAPY OF S. AUREUS CHRONIC INFECTIONS

| Treatment | # of Mammary Glands | % Cures | % Responding | # of Clear Milkings +/- SD[1] |
|---|---|---|---|---|
| 2mg IL-2(24)[2] | 12 | 9.1 (1) | 54.5[3] | 1.9 +/- 3.3 |
| 10mg IL-2(24)[2] | 12 | 25.0 (3) | 58.3 | 1.6 +/- 3.0 |
| 2mg IL-2(48)[4] | 11 | 8.3 (1) | 27.2[3] | 0.6 +/- 3.9 |
| 10mg IL-2(48)[4] | 12 | 25.0 (3) | 33.3 | 0.6 +/- 1.3 |

1 Number represents the mean of Staphylococcus aureus free milkings +/- the standard deviation, SD.

2 Treatment used 2 or 10 mg or r-BoIL-2 in a total volume of 10 ml of phosphate buffered saline infused into the infected mammary gland thrice at 24 hour intervals.

3 Percent of treated glands which demonstrated a bacteriological clearing of the infection for atleast one milking after treatment.

4 Treatment used 2 or 10 mg or r-BoIL-2 in a total volume of 10 ml of phosphate buffered saline infused into the infected mammary gland thrice at 48 hour intervals.

Example 10

Enhanced Efficacy of Mucolytic Proteins and Antibiotics with Chemoactivators

Mastitic quarters are treated with chemotractant and/or bactericidal agent, for example the mucolytic enzyme lysostaphin or an antibiotic. The lysostaphin is from a recombinant source and is administered at a dose of 100 mg as an intramammary infusion after three consecutive afternoon milkings of S. aureus infected quarters of dairy cattle. Similarly an antibiotic can be administered at doses ranging from 1 $\mu$g to 200 mg, 200 mg administered as an intramammary infusion of sodium cephapirin in saline after three consecutive afternoon milkings is preferred. The chemotractant, LPS (1$\mu$g X's 3), as a representative of the Class I type of chemoattractant/chemoactivator and ZACS (100% 10 ml X's 3), as a representative of Class II type chemoattractant are used. The combination of the bactericidal enzyme lysostaphin and the Class II chemoattractant does not improve the therapeutic efficacy of lysostaphin alone and may even hinder its efficacy (37.5% cures vs. 12.5% cures), Table VI. In contrast a Class I chemoattractant/chemoactivator, LPS, enhances the number of cures from 19.4% to 33.3%. The combination of Class I chemoattractant/chemoactivator with antibiotic and a bactericidal enzyme increases cures from 19.4% to 50%.

TABLE VI

COMBINATION THERAPY OF LYSOSTAPHIN AND CLASS I CHEMOACTIVATOR (ZACS)

| Treatment | Quarters | # of % Cures(#) | # of Clear Milkings ± SD[1] |
|---|---|---|---|
| ZACS[2] | 7 | 0.0 (0) | 0.0 ± 0.0 |
| Lysostaphin[3] | 8 | 37.5 (3) | 4.0 ± 1.32 |
| Lysostaphin + ZACS[4] | 8 | 12.5 (1) | 4.6 ± 1.34 |

1 Number represents the mean time of *S. aureus* free milkings ± the standard deviation, SD.

2 Treatment uses 10 ml of zymosan activated cow sera, ZACS, infused on three consecutive AM milkings. This dose elicits a significant quantitative influx of somatic cells within 24-72 hours in normal animals.

3 Treatment uses 100 mg of recombinant-lysostaphin infused on three consecutive PM milkings.

4 For the combination therapy of zymosan (10 ml) and recombinant-lysostaphin (100 mg), zymosan is infused on three consecutive AM milkings while recombinant-lysostaphin is infused during three consecutive PM milkings.

**TABLE VII**

**THERAPEUTIC SYNERGY OF BACTERICIDAL AGENTS AND CHEMOACTIVATORS**

| Treatment | # Quarters | % Cures(#) | Clear Milkings ± SD[1] |
|---|---|---|---|
| Lysostaphin (saline)[2] | 31 | 19.4 (6) | 7.3 ± 4.4 |
| Lysostaphin + LPS[3] | 27 | 33.3 (9) | 9.0 ± 5.1 |
| Lysostaphin/Cephapirin + LPS | 10 | 50.0 (5) | 9.0 ± 4.3 |
| Penicillin (saline) | 7 | 0.0 (0) | 5.8 ± 2.4 |
| Cephapirin (saline) | 17 | 29.4 (5) | 7.8 ± 3.2 |

1 Number represents the mean time of *S. aureus* free milkings +/- the standard deviation, SD.

2 Treatment used 100 mg of recombinant-lysostaphin infused on three consecutive PM milkings.

3 Treatment uses 1μg of lipopolysacharide, LPS, infused on three consecutive AM milkings. This dose elicits a significant quantitative influx of somatic cells in normal glands within 24-72 hours.

4 For the combination therapy of LPS (10ml) and recombinant-lysostaphin (100mg), LPS is infused on three consecutive AM milkings while recombinant-lysostaphin is infused during three consecutive PM milkings.

## Claims

1. Use of a phagocyte-activating agent lipopolysaccharide or interleukin-2 or lipopolysaccharide in combination with lysostaphin or cephapirin for the manufacture of a medicament for the treatment or prevention of Staphylococcus aureus infection in cows wherein said phagocyte-activating agent elicits an enhanced and sustained activation of phagocytic cells.

**2.** Use according to Claim 1 wherein said Staphylococcus aureus infection is mastitis.

**Patentansprüche**

**1.** Verwendung von Lipopolysaccharid oder Interleukin-2 oder Lipopolysaccharid in Verbindung mit Lysostaphin oder Cephapirin als Phagozyten-aktivierendes Mittel für die Herstellung eines Medikaments zur Behandlung oder Verhütung einer Staphylococcus aureus-Infektion bei Kühen, worin besagtes Phagozytenaktivierendes Mittel eine verstärkte und anhaltende Aktivierung von Phagozyten-Zellen hervorruft.

**2.** Verwendung nach Anspruch 1, bei der die Staphylococcus aureus-Infektion Mastitis ist.

**Revendications**

**1.** Utilisation du lipopolysaccharide ou de l'interleukine 2, ou du lipopolysaccharide en combinaison avec la lysostaphine ou la cépharine en tant qu'agent activateur de la phagocytose dans la préparation d'un médicament destiné au traitement ou à la prévention de l'infection à Staphylococcus aureus chez la vache, dans laquelle ledit agent activateur de la phagocytose induit une activation accrue et prolongée des cellules phagocytaires

**2.** Utilisation selon la revendication 1 dans laquelle ladite infection à Staphylococcus aureus est la mammite.

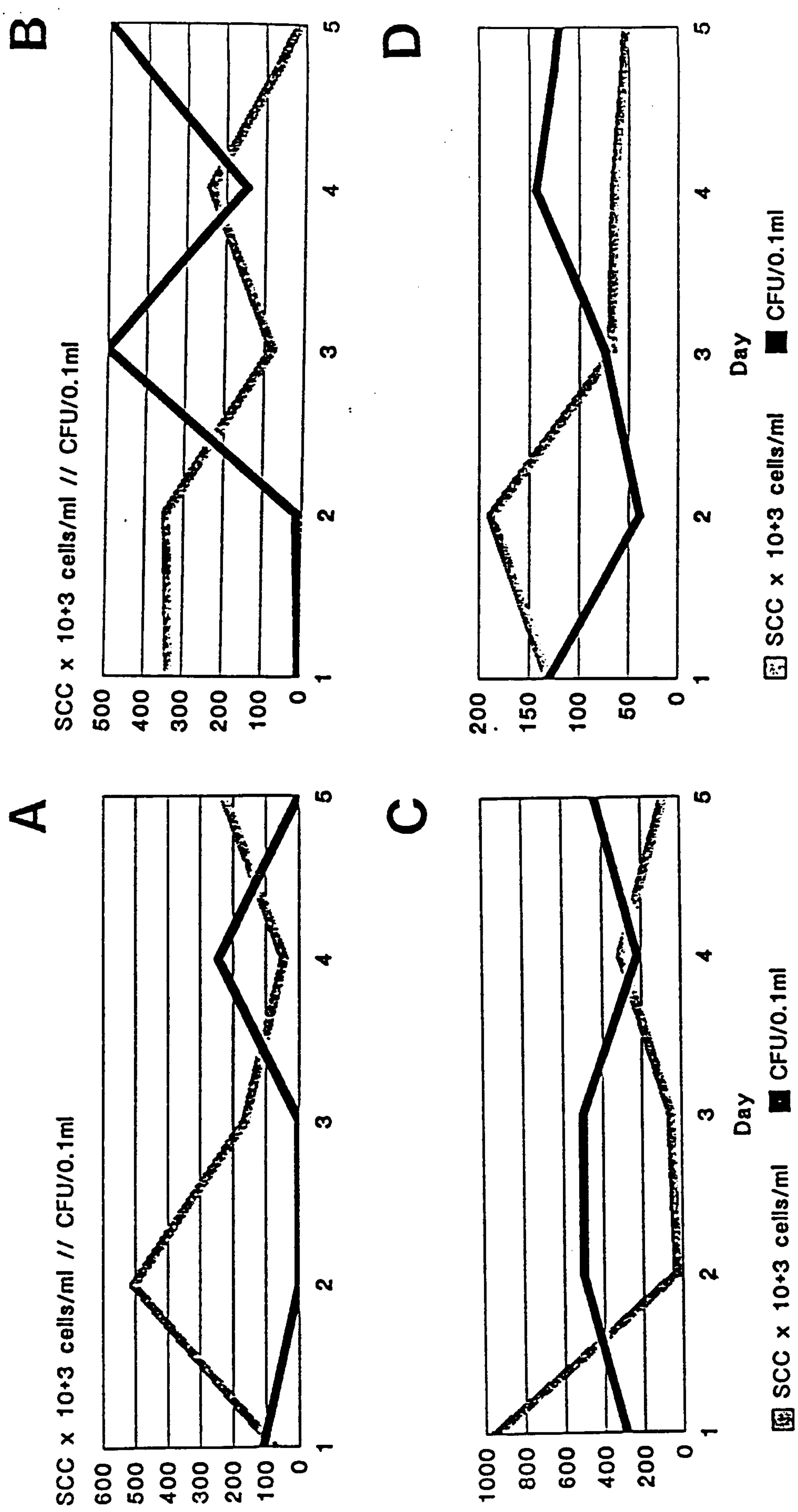
A
B
C
D
SCC x 10+3 cells/ml // CFU/0.1ml
SCC x 10+3 cells/ml // CFU/0.1ml
600 500 400 300 200 100 0
500 400 300 200 100 0
1000 800 600 400 200 0
200 150 100 50 0
1 2 3 4 5
Day
SCC x 10+3 cells/ml
CFU/0.1ml
Day
SCC x 10+3 cells/ml
CFU/0.1ml

# CHEMOTAXIS STUDY

## Effects of Various Chemotactic Factors on Milk Somatic Cells

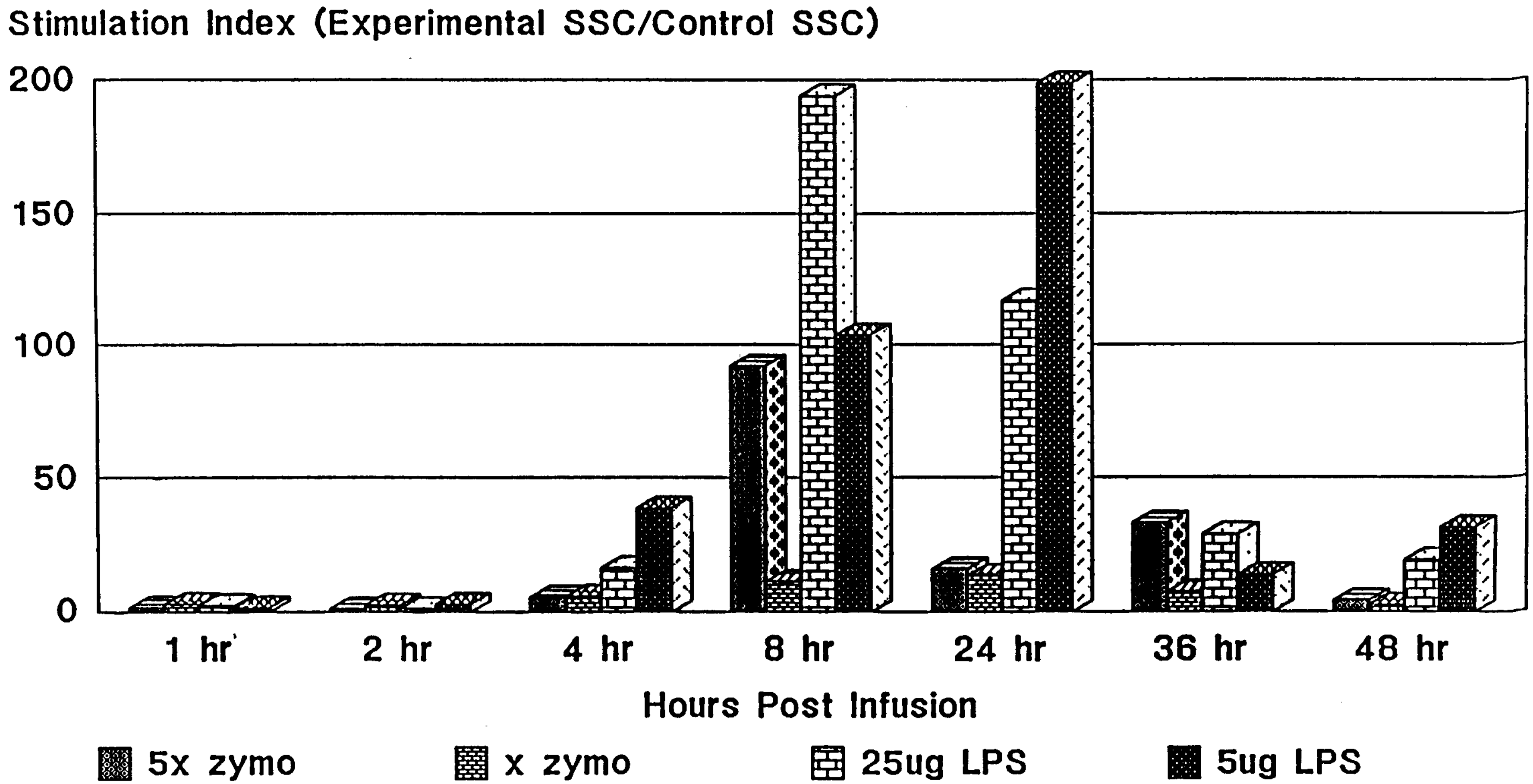

# CHEMOTAXIS STUDY

## Effects of Lipopolysaccharide on Milk Somatic Cells

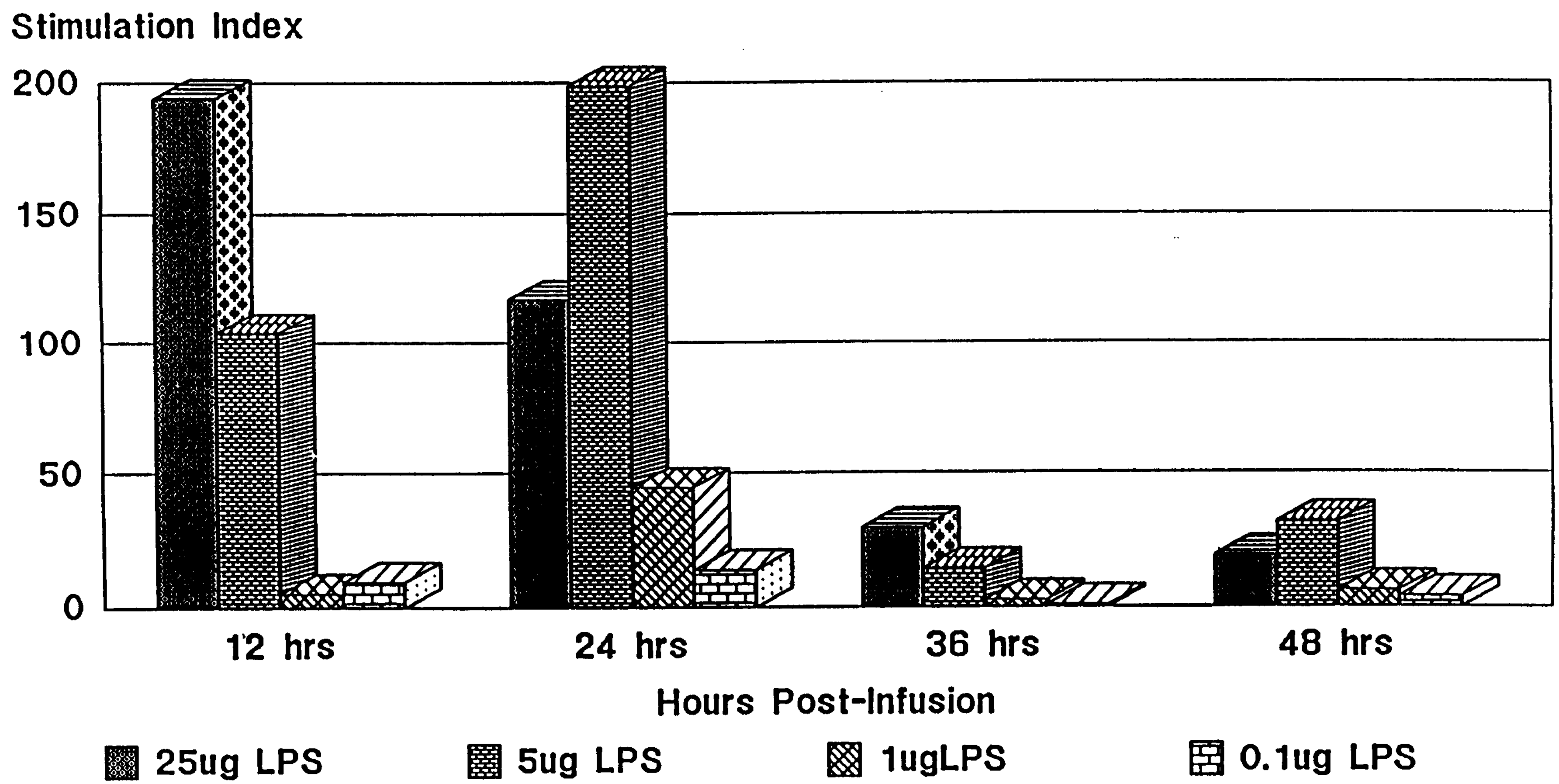

# CHEMOTRACTANT STUDY

## Lysostaphin Study

## Changes in Cell Diameter After Intramammary Infusion with Various Chemotractants

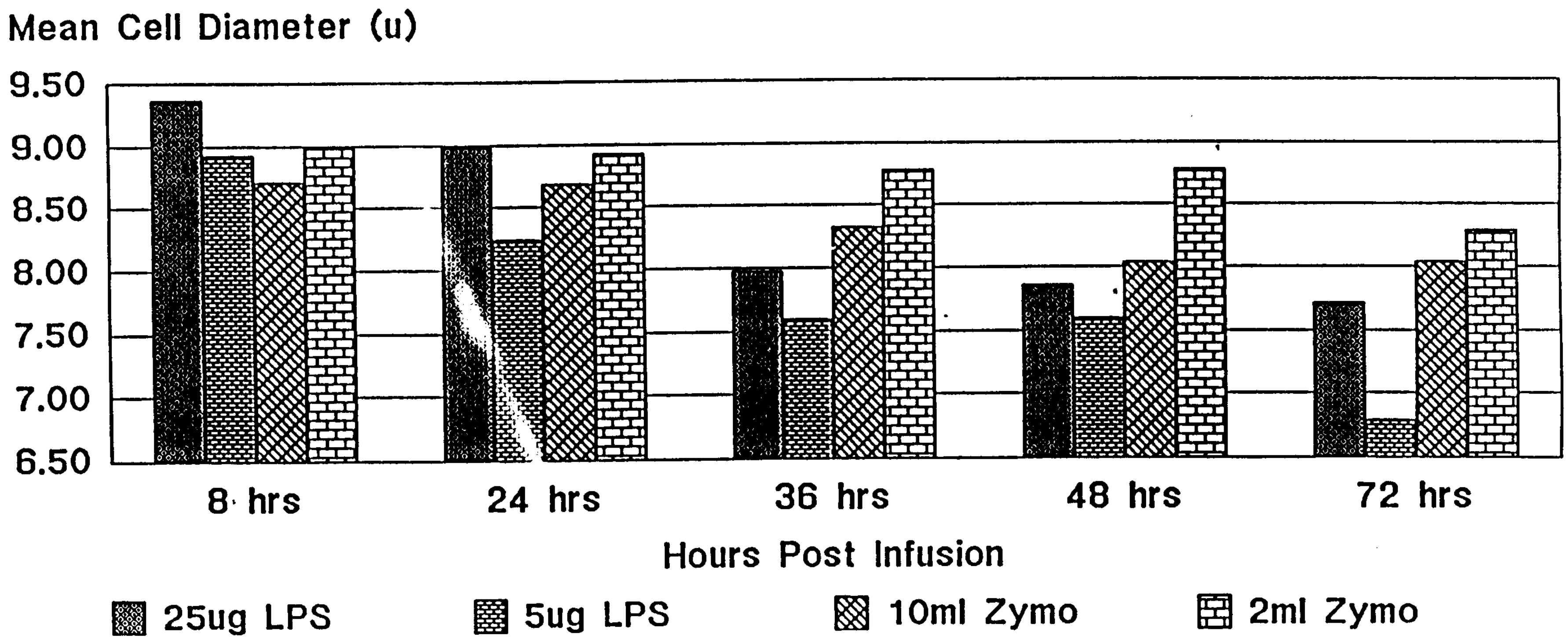

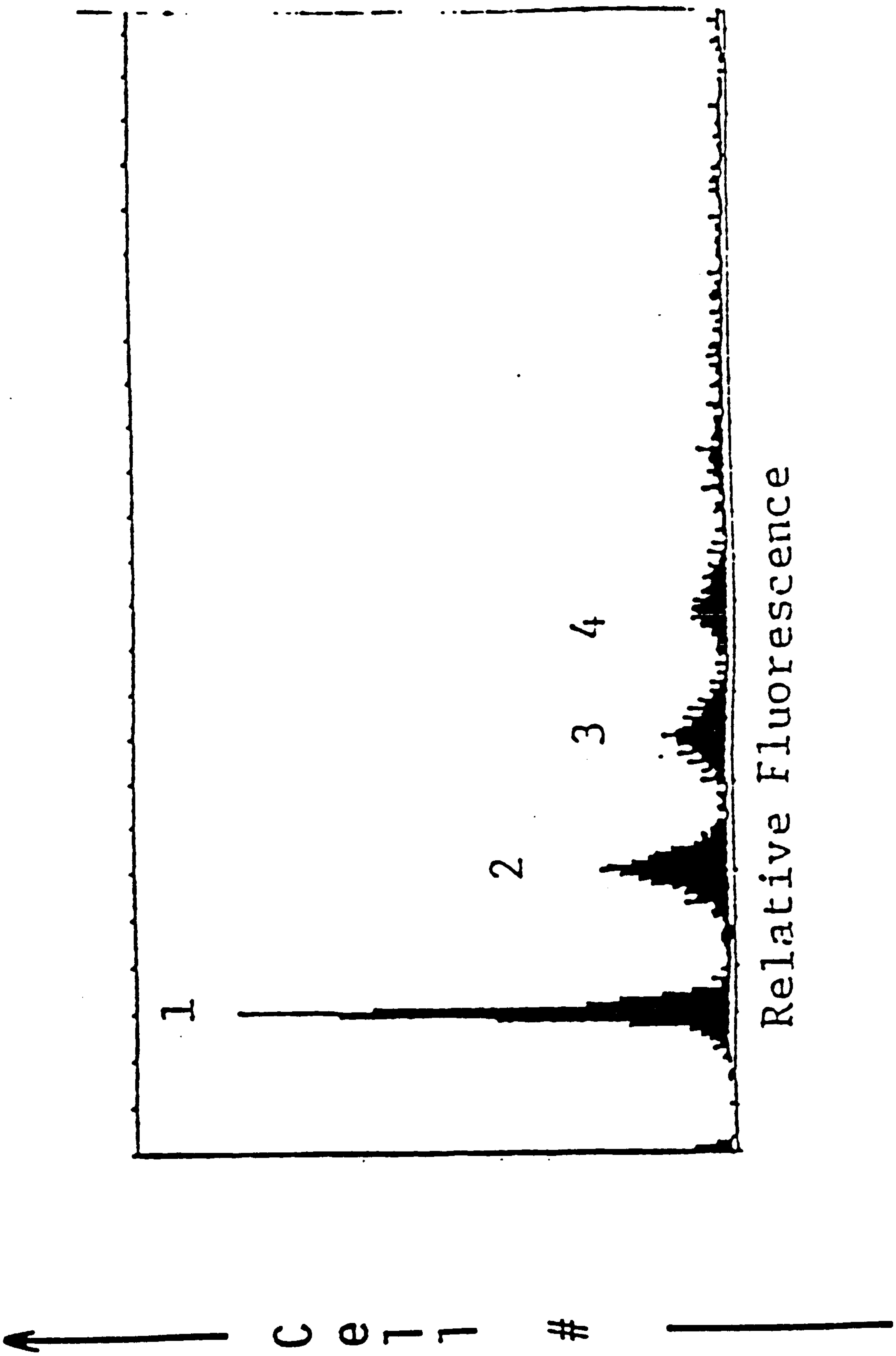
1
2
3
4
Relative Fluorescence
Cell #

# DOSE RESPONSE OF BOVINE MAMMARY GLAND TO RECOMBINANT BOVINE IL-2

IN NON-MASTITIC ANIMALS

## Single Intramammary Infusion - Response at 24 Hours

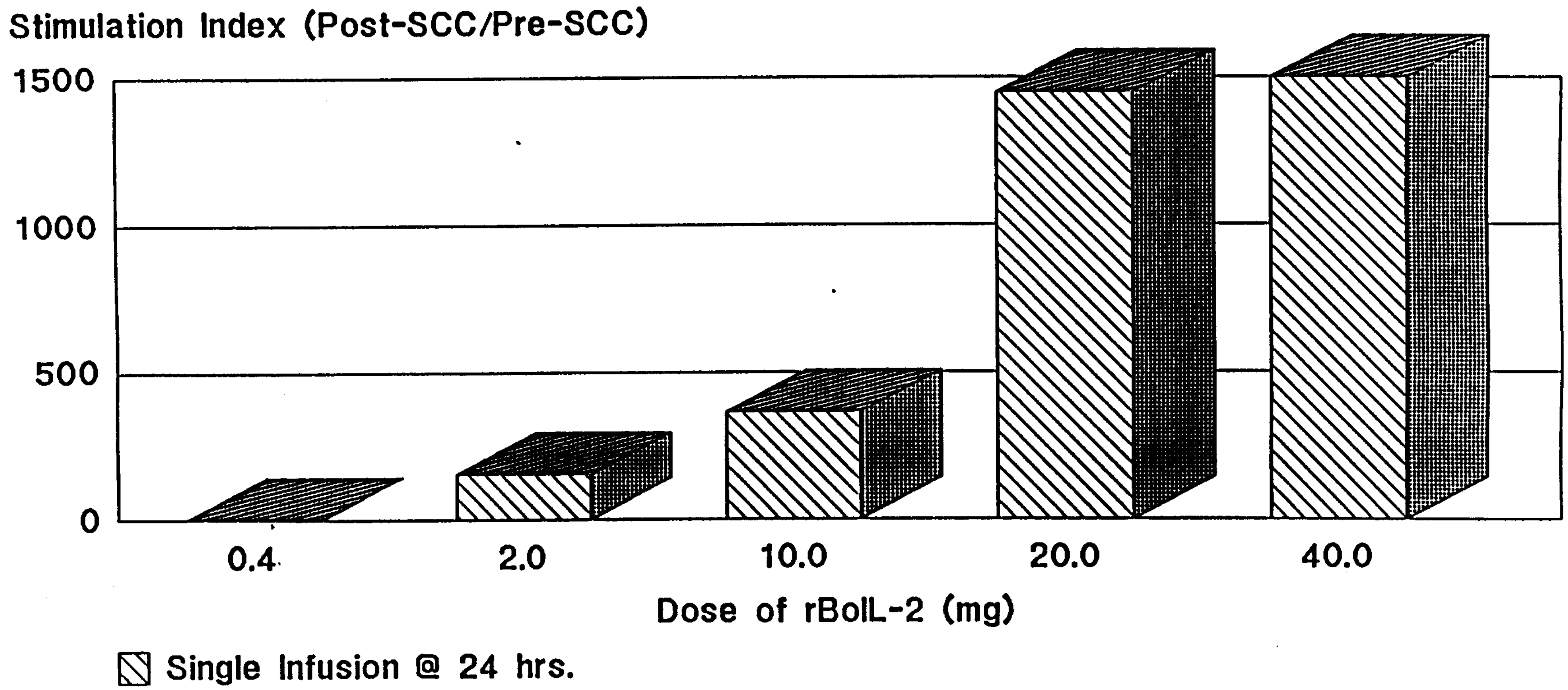

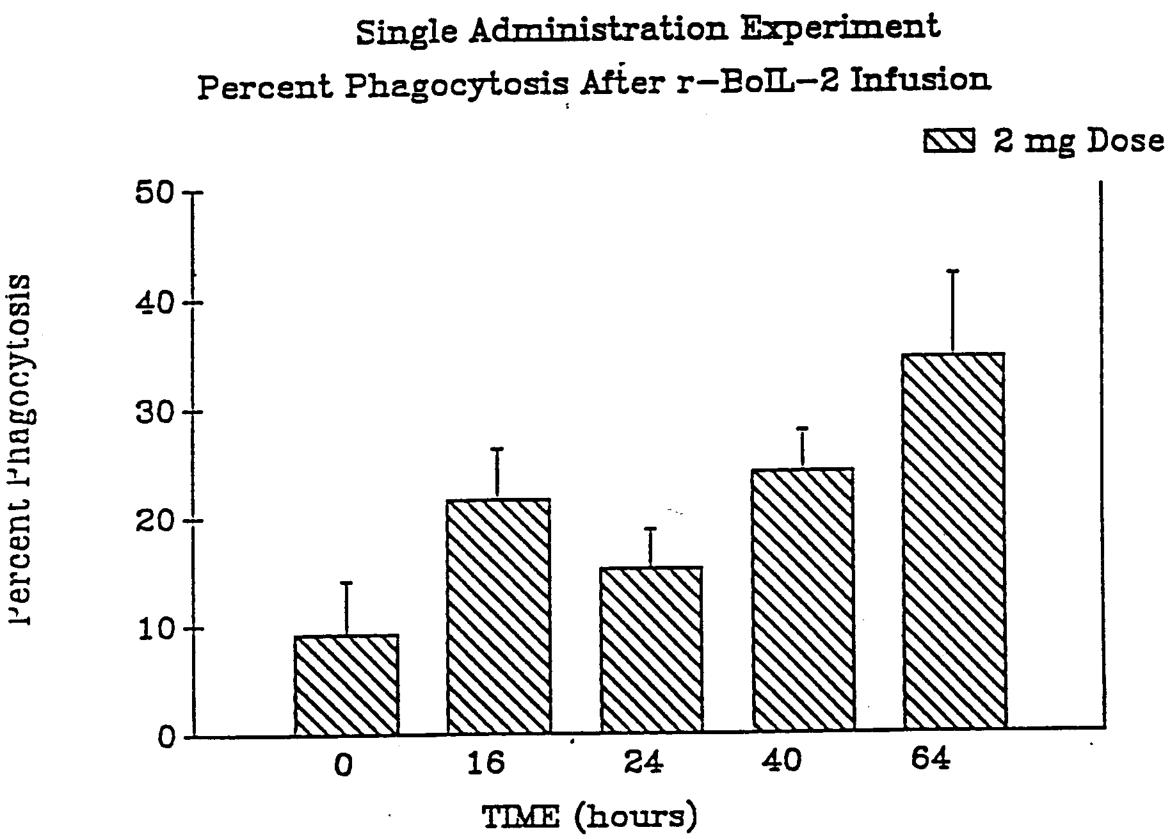
Single Administration Experiment
Percent Phagocytosis After r-BoIL-2 Infusion
2 mg Dose
Percent Phagocytosis
50
40
30
20
10
0
0
16
24
40
64
TIME (hours)

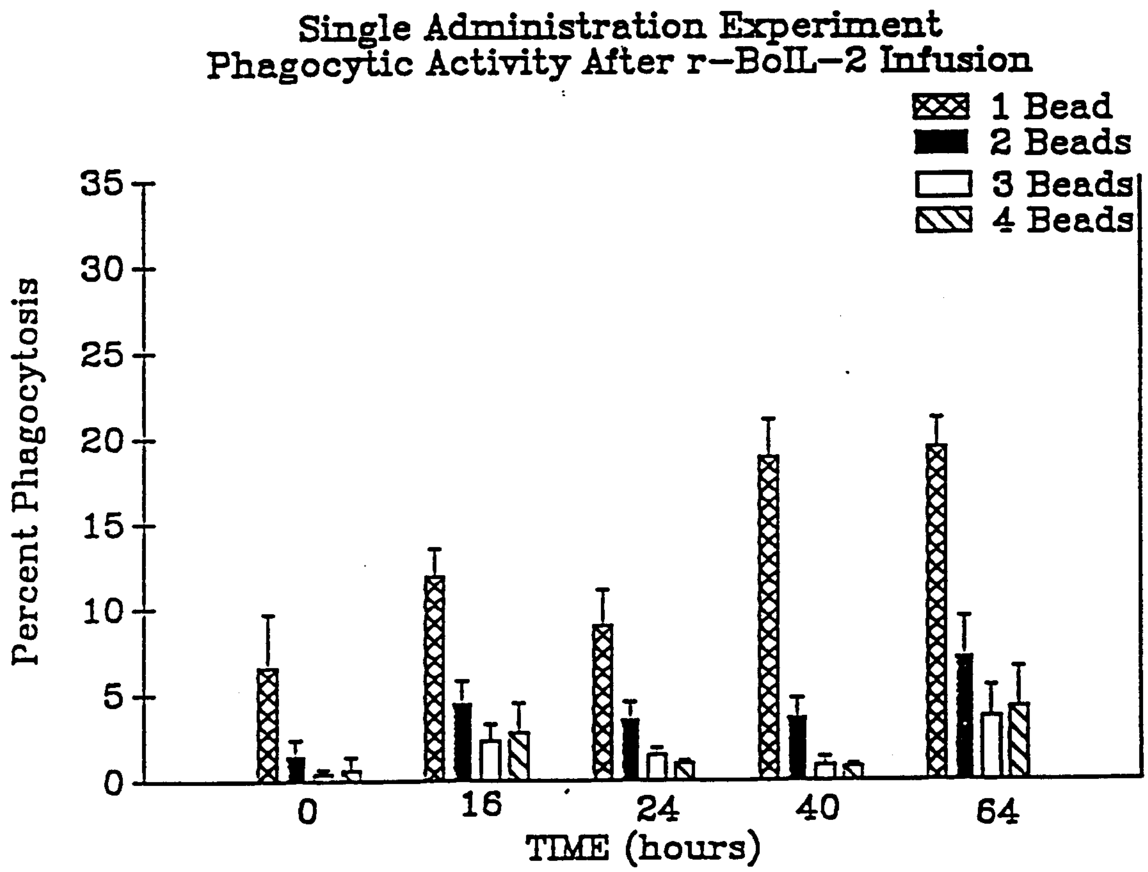
Single Administration Experiment
Phagocytic Activity After r–BoIL–2 Infusion
1 Bead
2 Beads
3 Beads
4 Beads
Percent Phagocytosis
0
5
10
15
20
25
30
35
0
16
24
40
64
TIME (hours)

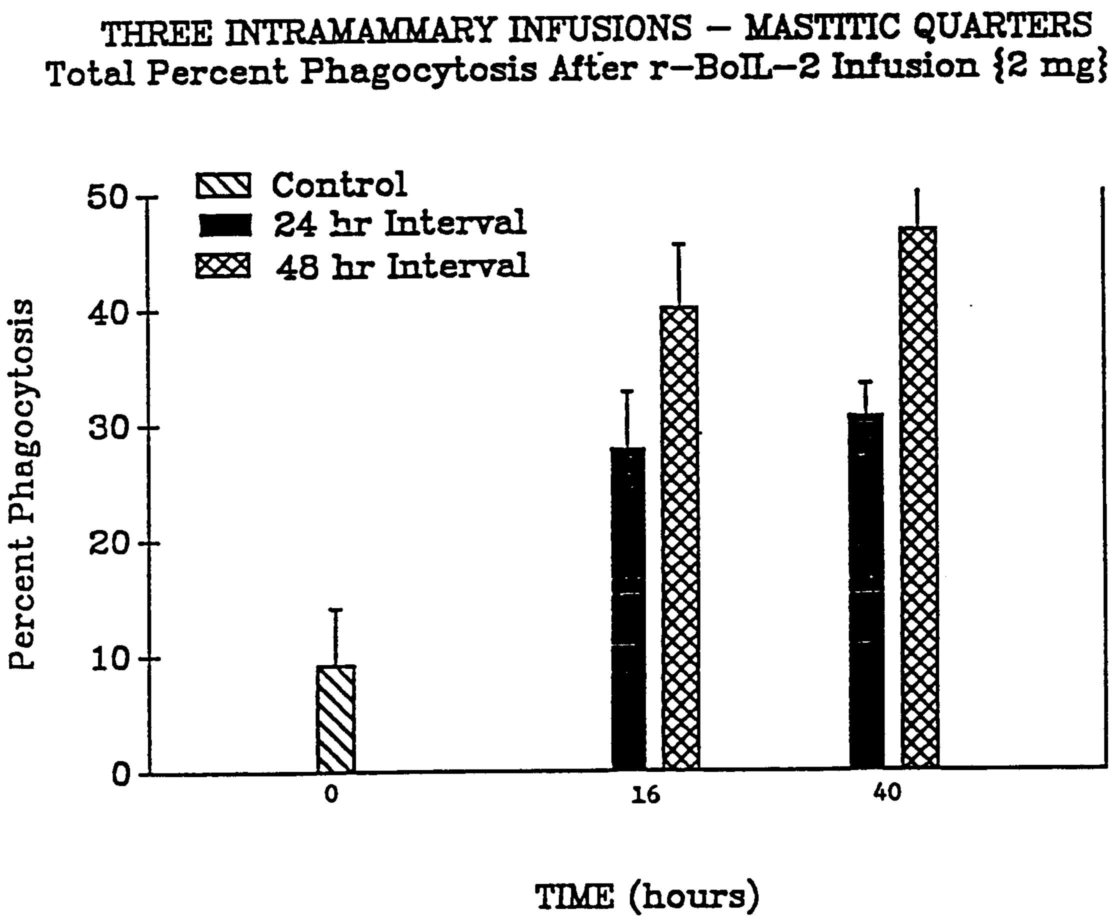
THREE INTRAMAMMARY INFUSIONS – MASTITIC QUARTERS
Total Percent Phagocytosis After r–BoIL–2 Infusion {2 mg}
Control
24 hr Interval
48 hr Interval
Percent Phagocytosis
0
10
20
30
40
50
0
16
40
TIME (hours)

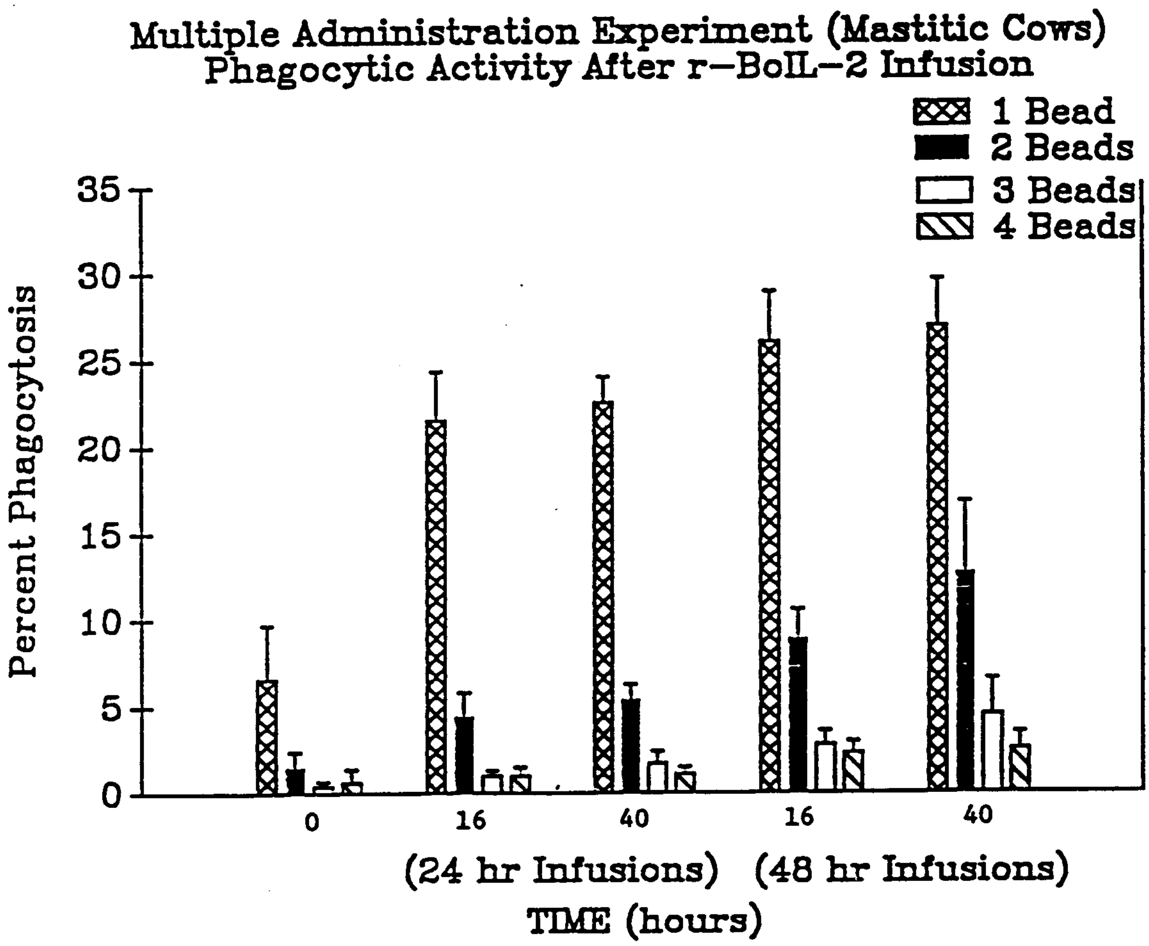
Multiple Administration Experiment (Mastitic Cows)
Phagocytic Activity After r-BoIL-2 Infusion
1 Bead
2 Beads
3 Beads
4 Beads
Percent Phagocytosis
35
30
25
20
15
10
5
0
0
16
40
16
40
(24 hr Infusions)
(48 hr Infusions)
TIME (hours)